(19) Europäisches Patentamt
European Patent Office
Office européen des brevets

(11) EP 1 704 814 B1

(12) **EUROPEAN PATENT SPECIFICATION**

(45) Date of publication and mention of the grant of the patent:
**29.10.2008 Bulletin 2008/44**

(51) Int Cl.:
***A61B 5/00*** ***(2006.01)***

(21) Application number: **06006317.9**

(22) Date of filing: **27.03.2006**

(54) **Optical tomography apparatus**

Gerät zur optischen Tomographie

Appareil pour la tomographie optique

(84) Designated Contracting States:
**DE FR GB**

(30) Priority: **25.03.2005 JP 2005089992**
**23.03.2006 JP 2006079933**

(43) Date of publication of application:
**27.09.2006 Bulletin 2006/39**

(73) Proprietors:
- **FUJIFILM Corporation**
  **Minato-ku**
  **Tokyo (JP)**
- **Fujinon Corporation**
  **Kita-ku**
  **Saitama-shi**
  **Saitama-ken (JP)**

(72) Inventors:
- **Toida, Masahiro**
  **c/o Fuji Photo Film Co.Ltd.**
  **Kanagawa-ken (JP)**
- **Tsujita, Kazuhiro**
  **c/o Fuji Photo Film Co.Ltd.**
  **Kanagawa-ken (JP)**
- **Fujita, Hiroshi**
  **c/o Fujinon Corporation**
  **Saitama-ken (JP)**

(74) Representative: **Klunker, Hans-Friedrich**
**Patentanwälte**
**Klunker . Schmitt-Nilson . Hirsch**
**Winzererstrasse 106**
**80797 München (DE)**

(56) References cited:
**WO-A-00/19889** **WO-A-01/50955**
**DE-A1- 19 853 669** **US-A1- 2003 055 342**
**US-A1- 2005 265 405**

- **S.H. YUN, G.J. TEARNEY, J.F. DE BOER, B.E. BOUMA: "Pulsed-Source and swept-source sprectral-domain optical coherence tomography with reduced motion artifacts" OPTICS EXPRESS, vol. 12, no. 23, 15 November 2004 (2004-11-15), pages 5614-5624, XP002401061 Optical Society of America**
- **B.B. DAS, FENG LIU, R.R. ALFANO: "Time-resolved fluorescence and photon migration studies in biomedical and model random media" REP. PROG. PHYS., vol. 60, 13 August 1996 (1996-08-13), pages 227-292, XP002401637 IOP Publishing Ltd.**
- **MICRON OPTICS: "ss225"[Online] XP002401058 Retrieved from the Internet: URL:http: //www.micronoptics.com/ss225.htm> [retrieved on 2006-09-28]**
- **SUPERLUM DIODES, LTD.: "SLD-53-MP"[Online] 3 February 2006 (2006-02-03), XP002401059 Retrieved from the Internet: URL: www.superlumdiodes.com/pdf/53mp.pdf> [retrieved on 2006-09-28]**
- **MEDOCT GRUPPE, UNIV. WIEN, AUSTRIA: "axiale Auflösung OCT"[Online] 2005, XP002401060 Wikipedia Retrieved from the Internet: URL:http://de.wikipedia.org/wiki/Bild: OCT-Aufl%C3%B6sung%C3%9CbersichtLichtquellen .p ng> [retrieved on 2006-09-28]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 704 814 B1

## Description

BACKGROUND OF THE INVENTION

### Field of the Invention

The present invention relates to an optical tomography apparatus that irradiates a light beam onto a measurement target to obtain tomographic images of the measurement target. Particularly, the present invention relates to an optical tomography apparatus that obtains images of the surface and the fine structures within the measurement target, based on a reflected light beam, which is the measuring light beam reflected by the measurement target.

### Description of the Related Art

As a conventional method for obtaining tomographic images of measurement targets, such as living tissue, a method that obtains optical tomographic images by TD-OCT (Time Domain Optical Coherence Tomography) measurement has been proposed (refer to Japanese Unexamined Patent Publication Nos. 6 (1994)-165784 and 2003-139688) . The TD-OCT measurement is a type of light interference measurement method that utilizes the fact that light interference is detected only when the optical path lengths of divided light beams, that is, a measurement light beam and a reference light beam, match within a range of coherence length of a light source. That is, in this method, a low coherent light beam emitted from a light source is divided into a measuring light beam and a reference light beam, the measuring light beam is irradiated onto a measurement target, and the measurement light beam reflected by the measurement target is led to a multiplexing means.

In the TD-OCT measurement, the measuring position (measuring depth) within the measurement target is changed, by changing the optical path length of either the reference light beam or the measuring light beam. Thereby, a one dimensional tomographic image in the direction of the optical axis is obtained. For example, the TD-OCT apparatus disclosed in Japanese Unexamined Patent Publication No. 6(1994)-165784 comprises an optical system that causes a reference light beam emitted from an optical fiber to be reflected by a mirror. The optical path length of the reference light beam is adjusted by moving the mirror in the direction of the optical axis of the reference light beam. In addition, the irradiation position of a measuring light beam, which is irradiated on a measurement target, is scanned in a direction perpendicular to the optical axis thereof, thereby enabling obtainment of two dimensional tomographic images based on two dimensional reflected optical intensities. Further, by scanning the irradiation position of the measuring light beam two dimensionally perpendicular to the optical axis thereof, three dimensional tomographic images can be obtained, based on three dimensional reflected optical intensities.

OCT apparatuses have been developed and are in use in the field of ophthalmology. Following the use of OCT apparatuses in the field of ophthalmology, research and development are underway for application in endoscopes. In the initial stages of development, the 0.8μm band had been employed as the wavelength of the light sources of the OCT apparatuses (refer to, for example, W. Drexler et al., "In Vivo Ultrahigh-Resolution Optical Coherence Tomography", Optics Letters, Vol. 24, No. 17, pp. 1221-1223, 1999.). This wavelength band was selected as a result of considering absorption properties of living tissue. Figure 1A is a graph that illustrates light absorption coefficients of water, blood, melanin, and epidermis. Figure 1B is a graph that illustrates the absorption coefficients of water with respect to light having wavelengths between 0.7μm and 1. 6μm. From the graph of Figure 1B, it can be seen that the peak of absorption occurs at 0.98μm and at 1.2μm. In addition, the broken line in the graph of Figure 2 is a graph that represents absorption loss in living tissue, based on the absorption coefficients. From the graph of Figure 2, it can be seen that light within the 0.8μm band has the smallest amount of absorption loss. For this reason, it was considered that light within the 0.8μm band has the highest transmissivity with respect to living tissue, enables deeper measurement depths, and is most suited for OCT apparatuses.

However, it has been found recently that scattering properties also limit measurement depths in OCT apparatuses. This is because OCT apparatuses detect backscattered reflected light beams from within living tissue. Rayleigh scattering is common within living tissue. In Rayleigh scattering, the scattering intensity is inversely proportionate to wavelength to the fourth power. The dotted line in the graph of Figure 2 represents scattering loss within living tissue. The total loss, represented by the solid line in the graph of Figure 2, is the sum of the absorption loss and the scattering loss.

From the graph of Figure 2, it can be seen that the wavelength band, at which total loss is minimal, is the 1.3μm band. For this reason, after OCT apparatuses for ophthalmology were realized, research and development for OCT apparatuses to be applied to endoscopes, which require deeper imaging depths, are being performed with the 1. 3μm band as the wavelength of light sources therein (refer to, for example, I. Hartl et al., "Ultrahigh-Resolution Optical Coherence Tomography Using Continuum Generation in an Air-Silica Microstructure Optical Fiber", Optics Letters, Vol. 26, No. 9, pp. 608-610, 2001. (and US 2003/055342).

The purpose for applying an OCT apparatus to an endoscope is to enable definitive diagnoses within living organisms, and to diagnose the depth of tumor invasion of mucosal cancer (m cancer) and submucosal cancer (sm cancer). Hereinafter, the procedure of endoscopic diagnosis of cancer will be briefly described. First, a diseased portion is discovered within a normal observation image, and whether the disease is cancer or another illness is discriminated. This preliminary diagnosis is based on the experience of a physician, after which tissue from a portion estimated to be cancerous is collected and subjected to a biopsy, to obtain a definitive diagnosis. For this reason, it is presently difficult to obtain definitive diagnoses during examination with an endoscope. In the case that a diseased portion is definitively diagnosed as cancer, the depth of tumor invasion is diagnosed by endoscopic examination, in order to determine a treatment strategy. Commonly, cancers present themselves in the mucoepidermis, and metastasize in the horizontal direction and in the depth direction, as the disease progresses. As illustrated in Figure 3, the structure of a stomach wall is constituted by: a membrana mucosa (m) layer; lamina muscularis mucosae (MM) ; a submucosal (sm) layer; tunica muscularis ventriculi; and a serous membrane. Cancers which are present only in the membrana mucosa layer are designated as m cancers, and cancers which have penetrated to the submucosal layer are designated as sm cancers. Treatment protocols differ between m cancers and sm cancers. Blood vessels and lymph systems are present in the submucosal layer, and there is a possibility of metastasis in the case of sm cancers. Therefore, surgical procedures are required. On the other hand, there is no possibility of metastasis in the case of m cancers. Therefore, m cancers are removed by endoscopic procedures. For this reason, it is necessary to discriminate whether cancers are m cancers or sm cancers. Specifically, it is important to be able to evaluate whether the layer structure of the lamina muscularis mucosae layer is maintained or destroyed, in an image. Presently, application of ultrasound imaging techniques is being considered, with the objective of diagnosing the depth of tumor invasion. However, the resolution of ultrasound imaging is only about 100μm in the axial direction, which is insufficient to visualize the MM layer. In addition, in m cancers which have progressed, lymph follicles are formed under the MM layer, thereby causing the cancerous portions and the lymph follicles to be imaged integrally, and m cancers may be misdiagnosed as sm cancers. For this reason, an imaging method having a resolution of 10μm or less in the axial direction is desired, to enable accurate diagnosis of the depth of tumor invasion.

Meanwhile, the resolution of an OCT apparatus in the optical axis direction is determined by the coherence length of the light source. That is, it is not generally possible to obtain resolution less than the coherence length of the light source. For this reason, a light beam having a coherence length of 10μm or less is necessary to obtain high resolution of 10μm or less. The coherence length Δz of low coherence light is proportionate to the square of the central frequency and inversely proportionate to the spectrum width thereof. The coherence length Δz can be expressed by the following formula:

$$\Delta z = (2\ln 2/\Pi) \cdot (\lambda c^2/\Delta\lambda)$$

wherein

λC: central wavelength
Δλ: spectrum width

For this reason, it is necessary to broaden the spectrum width Δλ in order to decrease the coherence length. However, it was found that the influence of dispersion needed to be considered, if the spectrum width Δλ was broadened (refer to Y. Wang et al., "Optimal Wavelength for Ultrahigh-Resolution Optical Coherence Tomography", Optics Express, Vol. 11, No. 12, pp. 1411-1417, 2003.).

In a Michaelson interferometer, as a light beam propagates through a sample, phase shift occurs, and a coherent signal waveform changes as a result. If the coherent signal waveform is designated as φ (w) and the spectrum waveform of the light source is a Gaussian distribution, autocorrelation functions can be expressed as:

$$\delta_t = \delta_{t0} \cdot \left\{ 1 + \left\langle \frac{d^2\varphi(w)}{dw^2} \right\rangle \delta w^4 \right\}^{\frac{1}{2}} \qquad (1)$$

$$K = \delta_t / \delta_{t0} \qquad (2)$$

$$D = \frac{-w_0^2}{2\pi c} \cdot \frac{d^2\varphi(w)}{dw^2} \quad (3)$$

wherein

$\delta_t$: $1/e^{1/2}$ width of the autocorrelation function
$\delta_{t0}$: $1/e^{1/2}$ width of the autocorrelation function when D=0
$\delta_w$: $1/e^{1/2}$ width of the optical spectrum
$w_0$: central frequency of the optical spectrum
K: broadening ratio due to the influence of dispersion

Figure 4 is a graph that illustrates calculated results (represented by the solid line) of formula (3) above and actual measured values (represented by the triangles) with water as the sample. Dispersion D is zero when the wavelength of the light beam is 1.0μm. It can be seen from the graph of Figure 4 that the influence of dispersion becomes greater as the wavelength becomes greater than or less than 1.0μm.

Figure 5 is a graph that illustrates simulation results of the relationship between the distance of propagation (depth of water) and broadening ratios, when low coherence light beams having wavelengths of 1. 32μm (spectrum width: 75nm), 1.2μm (spectrum width 62nm), 1.15μm (spectrum width: 59nm), and 0.98μm (spectrum width: 41nm) propagate through water. Note that the simulation results of Figure 5 are for a low coherence light beam having a central wavelength λc and a spectral width Δλ. However, the results can be applied to a coherent light beam, of which the wavelength is varied with a predetermined period, having a central wavelength λc and a wavelength sweep width Δλ.

In the aforementioned document, Y. Wang et al. conclude that it is preferable to employ low coherence light having a central wavelength of 1.0μm in OCT apparatuses, in the case that the coherence length of the low coherence light beam is short.

Meanwhile, in the aforementioned TD-OCT apparatus, the depth of positions at which measurement is performed is varied by moving a mirror, that is, by a mechanical means. Therefore, there is a problem that data collection takes a great amount of time.

Therefore, an OCT apparatus that utilizes a light source that emits a coherent light beam, of which the frequency is temporally varied, has been proposed (refer to, for example, U.S. patent No. 6,728,571.). In this OCT apparatus, coherent light is detected, and reflection intensities at depth positions within a measurement target are calculated, based on interferograms of optical frequency regions. Then, tomographic images are generated employing the calculated reflection intensities. This OCT apparatus would enable high speed obtainment of tomographic images, compared to an OCT apparatus that employs a low coherent light beam as measuring light and varies the measurement depth by moving a mirror.

The resolution in the optical axis direction is defined by the wavelength sweep width Δλ of the coherent light beam emitted by the light source in this SS-OCT apparatus as well. For this reason, the wavelength sweep width Δλ needs to be widened, in order to increase the resolution in the optical axis direction. However, if the wavelength sweep width Δλ is widened, it becomes necessary to consider the effects of scattering, as described above.

However, when an OCT apparatus that employs low coherence light is used to obtain an optical tomographic image of an organism, there are cases in which the wavelength band of the low coherence light (measuring light beam) includes wavelengths which are readily absorbed by living tissue. In these cases, the intensity of the reflected light beam is swept according to wavelength. As a result, pseudo signals are generated that reduce the S/N ratio of the optical tomographic image. As illustrated in Figure 1B, peaks in the absorption coefficient of water, which is the main constituent of living tissue, occur at wavelengths of 0.98μm and 1.2μm.

Note that in the aforementioned document by Y. Wang et al., it is disclosed that it is preferable to set the central wavelength of the measuring light beam in the vicinity of 1.0μm in cases that influence due to dispersion cannot be ignored, as a result of widening the wavelength range of the measuring light beam in order to obtain high resolution optical tomographic images. However, there is no disclosure regarding a central wavelength Δc nor a wavelength band width Δλ that avoids influence due to absorption at the 0.98μm and 1.2μm wavelengths.

SUMMARY OF THE INVENTION

The present invention has been developed in view of the aforementioned problems. It is an object of the present invention to clarify the presence of optimal wavelength sweep properties for obtaining high resolution while taking into consideration the light absorption properties, the scattering properties, and the dispersion properties of living organisms. It is another object of the present invention to realize an optical tomography apparatus that employs low coherence light, of which the wavelength is swept with a predetermined period within wavelengths of the optimal wavelength sweep properties to obtain high resolution optical tomographic images having high image quality.

The optical tomography apparatus of the present invention comprises:

a light source, for emitting a laser beam while sweeping through wavelengths at a predetermined period;
dividing means, for dividing the laser beam into a measuring light beam and a reference light beam;
an irradiating optical system, for irradiating the measuring light beam onto a measurement target;
multiplexing means, for multiplexing a reflected light beam, which is the measuring light beam reflected by the measurement target, and the reference light beam, to obtain a coherent light beam;
coherent light detecting means, for calculating the intensity of the reflected light beam at a plurality of depth positions within the measurement target, based on the frequency and the intensity of the coherent light beam; and
image obtaining means, for obtaining tomographic images of the measurement target, based on the intensities of the reflected light beam at each of the depth positions;
the central wavelength λc of the sweep and the wavelength sweep width Δλ of the laser light beam satisfying the following conditions:

$$\lambda c^2/\Delta\lambda \leq 23$$

$$\lambda c+(\Delta\lambda/2) \leq 1.2\mu m$$

$$\lambda c-(\Delta\lambda/2) \geq 0.98\mu m.$$

The central wavelength λc of the sweep and the wavelength sweep width Δλ of the laser light beam may satisfy the following condition:

$$\lambda c^2/\Delta\lambda \leq 17.$$

The coherent light detecting means may comprise an InGaAs type photodetector.

The optical tomography apparatus of the present invention comprises: a light source, for emitting a laser beam while sweeping through wavelengths at a predetermined period; dividing means, for dividing the laser beam into a measuring light beam and a reference light beam; an irradiating optical system, for irradiating the measuring light beam onto a measurement target; multiplexing means, for multiplexing a reflected light beam, which is the measuring light beam reflected by the measurement target, and the reference light beam, to obtain a coherent light beam; coherent light detecting means, for calculating the intensity of the reflected light beam at a plurality of depth positions within the measurement target, based on the frequency and the intensity of the coherent light beam; and image obtaining means, for obtaining tomographic images of the measurement target, based on the intensities of the reflected light beam at each of the depth positions. The central wavelength λc of the sweep and the wavelength sweep width Δλ of the laser light beam satisfies the following conditions: $\lambda c^2/\Delta\lambda \leq 23$; $\lambda c+(\Delta\lambda/2) \leq 1.2\mu m$; and $\lambda c-(\Delta\lambda/2) \geq 0.98\mu m$. Therefore, the transmissivity of the light beam is favorable, and the influence of light absorption having its peaks at the wavelengths 0.98μm and 1.2μm is reduced. Accordingly, high resolution optical tomographic images having high image quality can be obtained. In the case that the value of $\lambda C^2/\Delta\lambda$ is large, that is, the measurement resolution is low and the wavelength sweep width Δλ is narrow, there is almost no influence due to dispersion by water. However, when the value of $\lambda C^2/\Delta\lambda$ is small, the influence due to dispersion by water cannot be ignored.

From the simulation results of Figure 5, it can be seen that if the central wavelength λc is in the vicinity of 1.3μm, influence due to dispersion is observed even if $\lambda C^2/\Delta\lambda$ is approximately 23.

That is, it is considered that a light beam having a central wavelength of 1.0μm is superior to that having a central wavelength of 1.3μm, if the value of $\lambda C^2/\Delta\lambda$ is less than or equal to 23. Note that Figure 6 is a graph that represents central wavelengths λc and wavelength sweep widths Δλ that satisfy the above conditions.

In addition, it is considered that a light beam having a central wavelength of 1.0μm is superior to that having a central wavelength of 1.3μm, if the value of $\lambda C^2/\Delta\lambda$ is less than or equal to 17.

BRIEF DESCRIPTION OF THE DRAWINGS

Figure 1A is a graph that illustrates light absorption coefficients of water, blood, melanin, and epidermis.

Figure 1B is a graph that illustrates the absorption coefficients of water with respect to light having wavelengths between 0.7μm and 1.6μm.

Figure 2 is a graph for explaining absorption loss in living tissue, based on absorption coefficients.

Figure 3 is a diagram for explaining the progression of cancer in a stomach wall.

Figure 4 is a graph for explaining dispersion properties of water.

Figure 5 is a graph for explaining the relationship between distances of propagation in water and broadening ratios.

Figure 6 is a graph for explaining the relationship between central wavelengths and spectral bandwidths.

Figure 7 is a schematic diagram that illustrates the construction of an optical tomography apparatus according to an embodiment of the present invention.

Figure 8 is a graph for explaining a laser light beam La.

Figure 9 is a graph that illustrates the sensitivity of an InGaAs type photodetector.

DESCRIPTION OF THE PREFERRED EMBODIMENTS

Hereinafter, an optical tomography apparatus 100 according to a first embodiment of the present invention will be described with reference to Figure 7. Figure 7 is a schematic diagram that illustrates the construction of the optical tomography apparatus 100.

The optical tomography apparatus 100 illustrated in Figure 7 obtains tomographic images of measurement targets by the aforementioned SS-OCT measurement technique. The optical tomography apparatus 100 comprises: a light source unit 110, for emitting a laser light beam La; a light dividing means 3, for dividing the laser beam La into a measuring light beam L1 and a reference light beam L2; an optical path length adjusting means 120, for adjusting the optical path length of the reference light beam L2; an optical probe 130 that irradiates the measuring light beam L1 onto a measurement target S; a multiplexing means 4 (the light dividing means 3 functions as the multiplexing means 4), for multiplexing a reflected light beam L3, which is the measuring light beam L1 reflected from the measurement target S, and the reference light beam L2; and a coherent light detecting means 140, for detecting a coherent light beam L4, formed by multiplexing the reflected light beam L3 and the reference light beam L2.

The light source unit 110 emits the laser light beam La while sweeping the frequency thereof at a predetermined period. As illustrated in Figure 8, the frequency f of the laser light beam La is swept within a predetermined frequency sweep width Δf having a central frequency fc. Accordingly, the frequency F is swept in a saw blade pattern within the range of a frequency$_0$ (fc-Δf/2) to (fc+Δf/2).

Note that for the sake of simplicity in description, the variation in the frequency f of the laser light beam La will be described. However, the frequency f=light speed c/wavelength λ. Therefore, varying the frequency f of the laser light beam La at a predetermined period is equivalent to varying the wavelength λ of the laser light beam La. The central frequency fc illustrated in Figure 8 is the central wavelength λc when the wavelength λ is swept at the predetermined period, and thte frequency sweep width Δf is equivalent to a wavelength sweep width Δλ. In addition, Figure 8 illustrates an example in which the frequency is swept in a saw blade pattern. However, the frequency may be swept with any other waveform.

The central frequency fc and the frequency sweep width Δf are set such that the central wavelength λc and the wavelength sweep width Δλ of the laser light beam La satisfy the conditions:

$$\lambda c^2/\Delta\lambda \leq 23;$$

$$\lambda c+(\Delta\lambda/2)\leq 1.2\mu m;$$

and

$$\lambda c-(\Delta\lambda/2)\geq 0.98\mu m.$$

Note that the central frequency fc and the frequency sweep width Δf may be set such that the central wavelength λc and the wavelength sweep width Δλ of the laser light beam La satisfy the condition:

$$\lambda c^2/\Delta\lambda \leq 17.$$

The light source unit 110 comprises: a semiconductor optical amplifier 111 (semiconductor gainmedium) ; and an optical fiber FB10 . The optical fiber FB10 is connected to both ends of the semiconductor optical amplifier 111. The semiconductor optical amplifier 111 functions to emit a slight amount of light into a first end of the optical fiber FB10, when a drive current is injected thereinto, and to amplify the light that enters it from a second end of the optical fiber FB10. When the drive current is supplied to the semiconductor optical amplifier 111, the saw blade waveform laser light La is emitted to an optical fiber FB1 from an optical oscillator formed by the semiconductor optical amplifier 111 and the optical fiber FB10.

Further, an optical divider 112 is linked to the optical fiber FB10, and a portion of the light that propagates within the optical fiber FB10 is emitted into an optical fiber FB11. Light, which is emitted from the optical finer FB11, passes through a collimating lens 113, a diffraction grating 114, and an optical system 315, to be reflected by a rotating polygon mirror 116. The light reflected by the rotating polygon mirror 116 passes through an optical system 115, the diffraction grating 114, and the collimating lens 113, to reenter the optical fiber FB11.

The rotating polygon mirror 116 rotates in the direction indicated by arrow R1, to vary the angle of each reflective surface thereof with respect to the optical axis of the optical system 115. Thereby, only a light beam having a specific frequency, from among the light spectrally split by the diffraction grating 114, is returned to the optical fiber FB11. The frequency of the light beam that reenters the optical fiber FB11 is determined by the angle formed by the optical axis of the optical system 115 and the reflective surface of the rotating polygon mirror 116. The light that reenters the optical fiber FB11 is caused to enter the optical fiber FB10 by the optical divider 112. As a result, the laser light beam La of the specific frequency is emitted toward the optical fiber FB1.

Accordingly, when the rotating polygon mirror 116 is rotated in the direction of arrow R1 at a constant speed, the wavelength $\lambda$ of the light beam that reenters the optical fiber FB11 is varied over time, at a constant period. In this manner, the laser light beam La having the swept wavelengths is emitted to the optical fiber FB1 from the light source unit 110.

The light dividing means 3 is constituted by a 2x2 optical fiber coupler, for example. The light dividing means 3 functions to divide the light beam La, emitted by the light source unit 110 and guided through the optical fiber FB1, into a measuring light beam L1 and a reference light beam L2. The light dividing means 3 is optically connected to optical fibers FB2 and FB3. The measuring light beam L1 is guided through the optical fiber FB2, and the reference light beam L2 is guided through the optical fiber FB3. Note that the light dividing means 3 of the present embodiment also functions as the multiplexing means 4.

The optical probe 130 is to be inserted into body cavities via a forceps opening and a forceps channel, and is removably mounted to the optical fiber FB2 with an optical connector 31.

The optical probe 130 comprises: a probe outer cylinder 15, which has a closed distal end; a single optical fiber 13, which is provided to extend along the axial direction of the outer cylinder 15 within the interior space thereof; a prism mirror 17, for deflecting a light beam L emitted from the distal end of the optical fiber 15; a rod lens 18, for condensing the light beam L such that it converges on the measurement target S, which surrounds the outer cylinder 15; and a motor 14, for rotating the prism mirror 17 with the axis of the optical fiber 13 as the rotational axis.

The optical path length adjusting means 120 is provided at the end of the optical fiber FB3 at which the reference light beam L2 is emitted. The optical path length adjusting means 120 functions to change the optical path length of the reference light beam L2, to adjust the position at which tomographic images are obtained. The optical path length adjusting means 220 comprises: a mirror 22, for reflecting the reference light beam L2 emitted from the optical fiber FB3; a first optical lens 21a, provided between the optical fiber FB3 and the mirror 22; and a second optical lens 21b, provided between the first optical lens 21a and the mirror 22.

The first optical lens 21a functions to collimate the reference light beam L2 emitted from the optical fiber FB3, and to focus the reference light beam L2 reflected by the mirror 22 onto the core of the optical fiber FB3. The second optical lens 21b functions to focus the reference light beam L2 collimated by the first optical lens 21a onto the mirror 22, and to collimate the reference light beam L2 reflected by the mirror 22. That is, the first optical lens 21a and the second optical lens 21b form a confocal optical system.

Accordingly, the reference light beam L2 emitted from the optical fiber FB3 is collimated by the first optical lens 21a, and focused on the mirror 22 by the second optical lens 21b. Thereafter, the reference light beam L2 reflected by the mirror 22 is collimated by the second optical lens 21b, and focused onto the core of the optical fiber FB3.

The optical path length adjusting means 120 further comprises: a base 23, on which the second optical lens 21b and the mirror 22 are fixed; and a mirror moving means 24, for moving the base 23 in the direction of the optical axis of the first optical lens 21a. The optical path length of the reference light beam L2 is varied, by moving the base 23 in the direction indicated by arrow A.

The multiplexing means 4 is constituted by the aforementioned 2x2 optical coupler. The multiplexing means 4 multiplexes the reference light beam L2, of which the frequency has been shifted and the optical path length has been adjusted by the optical path length adjusting means 120, and the reflected light beam L3 reflected by the measurement target S. The multiplexed coherent light beam L4 is emitted toward the coherent light detecting means 140 via the optical fiber FB4.

The coherent light detecting means 140 detects the coherent light beam L4, and measures the intensity thereof. The coherent light detecting means 240 comprises: InGaAs type photodetectors 40a and 40b, for measuring the intensity of the coherent light beam L4; and a calculating section 141, for adjusting the input balance of detection values obtained by the photodetectors 40a and 40b, to enable balanced detection. Note that the coherent light beam L4 is divided into two light beams by the light divided means 3, and the divided light beams are detected by the photodetectors 40a and 40b, respectively.

An image obtaining means 150 administers Fourier transform on the coherent light beam L4 detected by the coherent light detecting means 140 to calculate the intensity of the reflected light beam L3 at each depth position within the measurement target S. Thereby, tomographic images of the measurement target S are obtained. The obtained tomographic images are displayed by a display apparatus 160.

Here, detection of the coherent light beam L4 by the coherent light detecting means 140 and image generation by the image obtaining means 150 will be described briefly. Note that a detailed description of these two points can be found in M. Takeda, "Optical Frequency Scanning Interference Microscopes", Optical and Electro-Optical Engineering Contact, Vol. 41, No. 7, pp. 426-432, 2003.

When the measuring light beam L1 is irradiated onto the measurement target S, the reflected light beams L3, which are reflected at various depths within the measurement target S and the reference light beam L2 interfere with each other, with various optical path length differences. By designating the optical intensity of the interference pattern with respect to each of the optical path length differences 1 as S(l), the optical intensity I(k) detected by the coherent light detecting means 140 can be expressed as:

$$I(k)=\int_0^{\infty} S(l)\,[1+\cos(kl)]\,dl$$

wherein:

k: wave number
l: optical path length difference

The above formula may be considered as being provided as an interferogram of an optical frequency range, in which the wave number k=ω/c is a variable. For this reason, the image obtaining means 250 administers Fourier transform on the spectral interference pattern detected by the coherent light detecting means 140, to determine the optical intensity (l) of the coherent light beam L4. Thereby, data regarding the distance from a measuring position within the measurement target S and data regarding the intensity of the reflected light beam can be obtained, and generation of tomographic images is enabled.

Hereinafter, the operation of the optical tomography apparatus 100 of the above construction will be described. When obtaining a tomographic image, first, the base 23 is moved in the direction of arrow A, to adjust the optical path length such that the measurement target S is positioned within a measurable region. Thereafter, the light beam La is emitted from the light source unit 110. The light beam La is divided into the measuring light beam L1 and the reference light beam L2 by the light dividing means 3. The measuring light beam L1 is emitted within the body cavity from the optical probe 130, and irradiated on the measurement target S. At this time, the measuring light beam L1 scans the measurement target S one dimensionally, by the optical probe 130 operating as described above. The reflected light beam L3, reflected by the measurement target S, is multiplexed with the reference light beam L2, reflected by the mirror 22, to form the coherent light beam L4. The coherent light beam L4 is detected by the coherent light detecting means 140. The image obtaining means 150 administers appropriate waveform compensation and noise removal on the detected coherent light beam L4, then administers Fourier transform thereon, to obtain intensity distribution data of the reflected light in the depth direction of the measurement target.

Next, the motor 14 of the optical probe 130 rotates the prism mirror 17, thereby scanning the measuring light beam L1 on the measurement target S. Thereby, data regarding each portion along the scanning direction can be obtained, and a tomographic image of tomographic sections that include the scanning direction can be obtained. The tomographic image obtained in this manner is displayed by the display apparatus 160. Note that by moving the optical probe 130 in the horizontal direction in Figure 7, the measuring light beam L1 can be scanned in a second direction perpendicular to the aforementioned scanning direction. Thereby, a tomographic image of tomographic sections that include the second direction can be further obtained. The tomographic image obtained in this manner is also displayed by the display apparatus 160.

The central wavelength λc and the wavelength sweep width Δλ of the laser light beam La satisfy the condition:

$$\lambda c^2/\Delta\lambda \leq 23.$$

Therefore, a laser light beam having a central wavelength in the 1.0μm band is superior to that having a central wavelength in the 1.3μm band.

Further, the conditions:

$$\lambda c + (\Delta\lambda/2) \leq 1.2\mu m$$

$$\lambda c - (\Delta\lambda/2) \geq 0.98\mu m$$

are satisfied. Therefore, the measuring light beam L1 has good transmissivity with respect to the measurement target S, and the influence exerted on the reflected light beam L3 by the light absorption peaks of water at the wavelengths of 0.98μm and 1.2μm is decreased. Accordingly, high resolution optical tomographic images having high image quality can be obtained.

Note that a laser light beam La may be employed, in which the central wavelength λc and the wavelength sweep width Δλ of the laser light beam La satisfy the condition:

$$\lambda c^2/\Delta\lambda \leq 17.$$

In this case, a laser light beam having a central wavelength in the 1.0μm band would be particularly superior to that having a central wavelength in the 1.3μm band.

Note that in the case that the central wavelength λc of the laser light beams La is greater than or equal to 0.98μm and less than or equal to 1.2μm, it is preferable that InGaAs type photodetectors are employed as the photodetectors 40a and 40b, as in the present embodiment. As illustrated by the sensitivity properties illustrated in the graph of Figure 9, InGaAs photodetectors can positively detect the coherent light beam L4.

Note that the optical tomography apparatus of the present invention is not limited to the embodiment described above. For example, the optical tomography apparatus 1 illustrated in Figure 7 exemplifies a case in which the laser light beam La, the measuring light beam L1, the reference light beam L2, the reflected light beam L3, and the coherent light beam L4 propagate through optical fibers. Alternatively, the light beams may propagate through air or through a vacuum.

## Claims

**1.** An optical tomography apparatus (100), comprising:

a light source (110), for emitting a laser beam (La) while sweeping through wavelengths at a predetermined period;
dividing means (3), for dividing the laser beam (La) into a measuring light beam (L1) and a reference light beam (L2);
an irradiating optical system, for irradiating the measuring light beam onto a measurement target (S);
multiplexing means (4), for multiplexing a reflected light beam (L3), which is the measuring light beam (L1) reflected by the measurement target (S), and the reference light beam (L2), to obtain a coherent light beam (L4);
coherent light detecting means (140), for calculating the intensity of the reflected light beam (L3) at a plurality of depth positions within the measurement target (S), based on the frequency and the intensity of the coherent light beam (L4); and
image obtaining means (150), for obtaining tomographic images of the measurement target (S), based on the intensities of the reflected light beam (L3) at each of the depth positions;
**characterised by** the central wavelength λc of the sweep and the wavelength sweep width Δλ of the laser light beam (La) satisfying the following conditions:

$$\lambda c^2/\Delta\lambda \leq 23$$

$$\lambda c+(\Delta\lambda/2)\leq 1.2\mu m$$

$$\lambda c-(\Delta\lambda/2)\geq 0.98\mu m.$$

**2.** An optical tomography apparatus (100) as defined in Claim 1, wherein:

the central wavelength λc of the sweep and the wavelength sweep width Δλ of the laser light beam (La) satisfy the following condition:

$$\lambda c^2/\Delta\lambda\leq 17.$$

**3.** An optical tomography apparatus as defined in either one of Claims 1 or 2, wherein:

the coherent light detecting means (140) comprises an InGaAs type photodetector (40a, 40b).

**Patentansprüche**

**1.** Optische Tomographievorrichtung (100), umfassend:

eine Lichtquelle (110) zum Emittieren eines Laserstrahls (La) bei Wobbeln über Wellenlängen mit einer vorbestimmten Periodendauer;
eine Teilereinrichtung (3) zum Teilen des Laserstrahls (La) in einen Messlichtstrahl (L1) und einen Referenzlichtstrahl (L2);
eine Aufstrahloptik zum Aufstrahlen des Messlichtstrahls auf ein Messziel (S);
eine Multiplexeinrichtung (4) zum Multiplexen eines reflektierten Lichtstrahls (L3), bei dem es sich um den von dem Messziel (S) reflektierten Messlichtstrahl (L1) handelt, und des Referenzlichtstrahls (L2), um einen kohärenten Lichtstrahl (L4) zu erhalten;
eine Kohärenzlicht-Detektoreinrichtung (140) zum Berechnen der Intensität des reflektierten Lichtstrahls (L3) an mehreren Tiefenstellen innerhalb des Messziels (S), basierend auf der Frequenz und der Intensität des kohärenten Lichtstrahls (L4); und
eine Bildgewinnungseinrichtung (150) zum Gewinnen von Tomographiebildern des Messziels (S) basierend auf den Intensitäten des reflektierten Lichtstrahls (L3) an jeder der Tiefenstellen;
**dadurch gekennzeichnet, dass** die Mittenwellenlänge λc der Wobbelung und der Wellenlängenhub Δλ des Laserlichtstrahls (La) folgende Bedingungen erfüllen:

$$\lambda c^2/\Delta\lambda \leq 23$$

$$\lambda c+(\Delta\lambda/2) \leq 1{,}2\ \mu m$$

$$\lambda c-(\Delta\lambda/2) \geq 0{,}98\ \mu m.$$

**2.** Vorrichtung (100) nach Anspruch 1, bei der die Mittenwellenlänge λc der Wobbelung und der Wellenlängenhub Δλ des Laserlichtstrahls (La) folgende Bedingung erfüllt:

$$\lambda c2/\Delta\lambda \leq 17.$$

**3.** Vorrichtung nach Anspruch 1 oder 2, bei der die Kohärenzlicht-Detektoreinrichtung (140) einen InGaAs-Photodetektor (40a, 40b) enthält.

## Revendications

**1.** Appareil pour la tomographie optique (100), comprenant :

une source de lumière (110), pour émettre un faisceau de lumière laser (La) tout en effectuant un balayage entre des longueurs d'onde à une période de temps prédéterminée ;
des moyens de division (3), pour diviser le faisceau de lumière laser (La) en un faisceau de lumière de mesure (L1) et un faisceau de lumière de référence (L2) ;
un système optique d'irradiation, pour irradier le faisceau de lumière de mesure sur une cible de mesure (S) ;
des moyens de multiplexage (4), pour multiplexer un faisceau de lumière réfléchie (L3), qui correspond au faisceau de lumière de mesure (L1) réfléchi par la cible de mesure (S) et au faisceau de lumière de référence (L2), de façon à obtenir un faisceau de lumière cohérente (L4) ;
des moyens de détection de lumière cohérente (140), pour calculer l'intensité du faisceau de lumière réfléchie (L3) à une pluralité de positions de profondeur à l'intérieur de la cible de mesure (S), sur la base de la fréquence et de l'intensité du faisceau de lumière cohérente (L4) ; et
des moyens d'obtention d'images (150), pour obtenir des images tomographiques de la cible de mesure (S), sur la base de l'intensité du faisceau de lumière réfléchie (L3) à chacune des positions de profondeur ;
**caractérisé en ce que** la longueur d'onde centrale λc du balayage, et la largeur de balayage en longueur d'onde Δλ du faisceau de lumière laser (La) satisfont les conditions suivantes :

$$\lambda c^2 / \Delta\lambda \leq 23$$

$$\lambda c + (\Delta\lambda / 2) \leq 1{,}2\ \mu m$$

$$\lambda c - (\Delta\lambda / 2) \geq 0{,}98\ \mu m$$

**2.** Appareil pour la tomographie optique (100) selon la revendication 1, dans lequel :

la longueur d'onde centrale λc du balayage, et la largeur de balayage en longueur d'onde Δλ du faisceau de lumière laser (La) satisfont la condition suivants :

$$\lambda c^2 / \Delta\lambda \leq 17$$

**3.** Appareil pour la tomographie optique selon l'une quelconque des revendications 1 ou 2, dans lequel ; les moyens de détection de lumière cohérente (140) comprennent un photodétecteur du type InGaAs (40a, 40b).

# FIG.1A

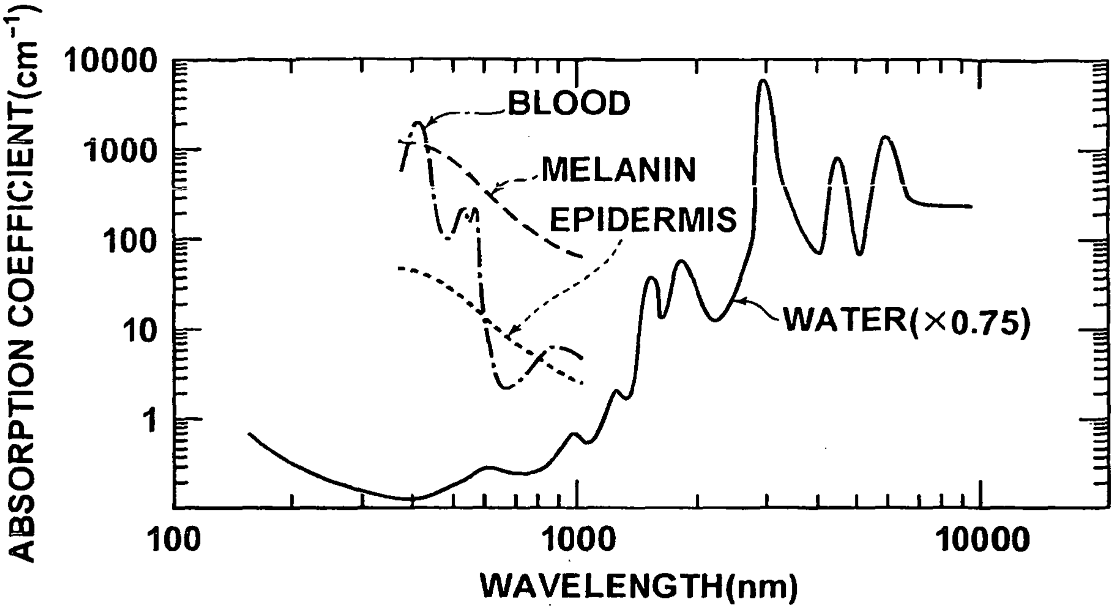
ABSORPTION COEFFICIENT(cm-1)
10000
1000
100
10
1
BLOOD
MELANIN
EPIDERMIS
WATER(×0.75)
100
1000
10000
WAVELENGTH(nm)

# FIG.1B

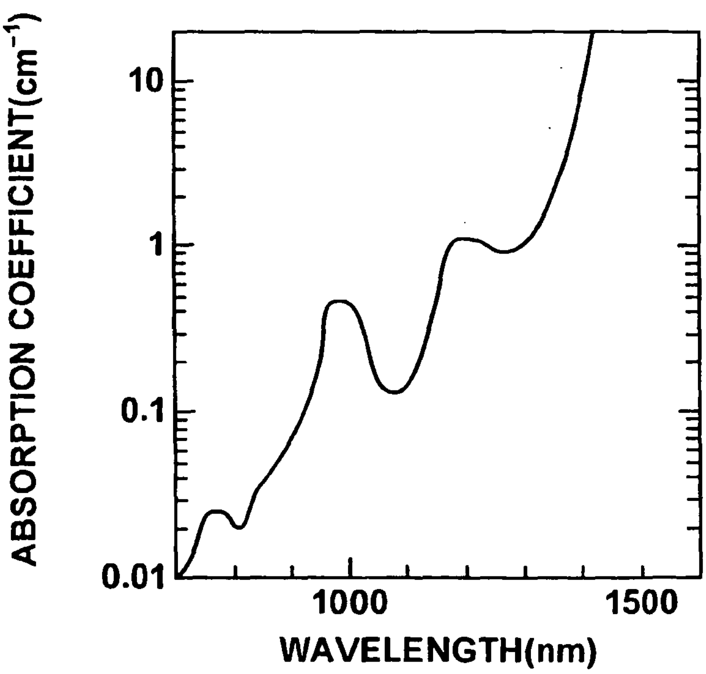
ABSORPTION COEFFICIENT(cm-1)
10
1
0.1
0.01
1000
1500
WAVELENGTH(nm)

# FIG.2

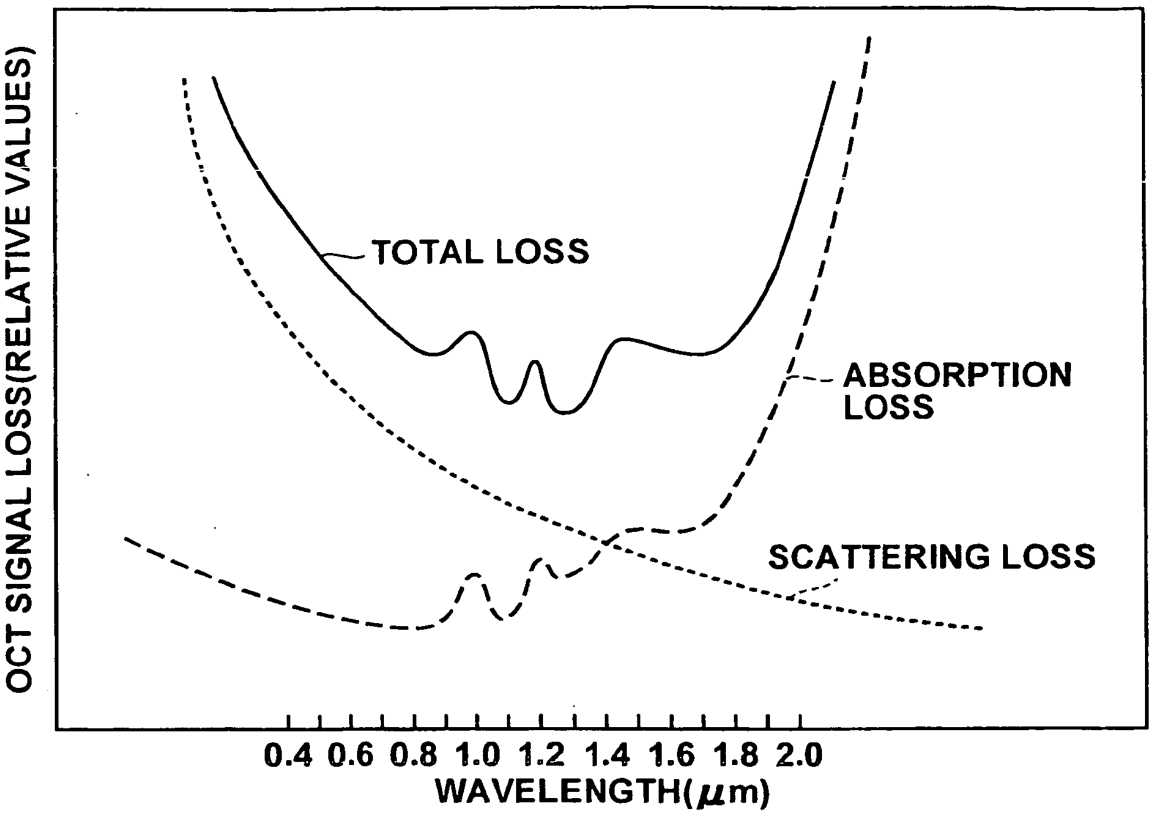
OCT SIGNAL LOSS(RELATIVE VALUES)
TOTAL LOSS
ABSORPTION LOSS
SCATTERING LOSS
0.4 0.6 0.8 1.0 1.2 1.4 1.6 1.8 2.0
WAVELENGTH(μm)

# FIG.3

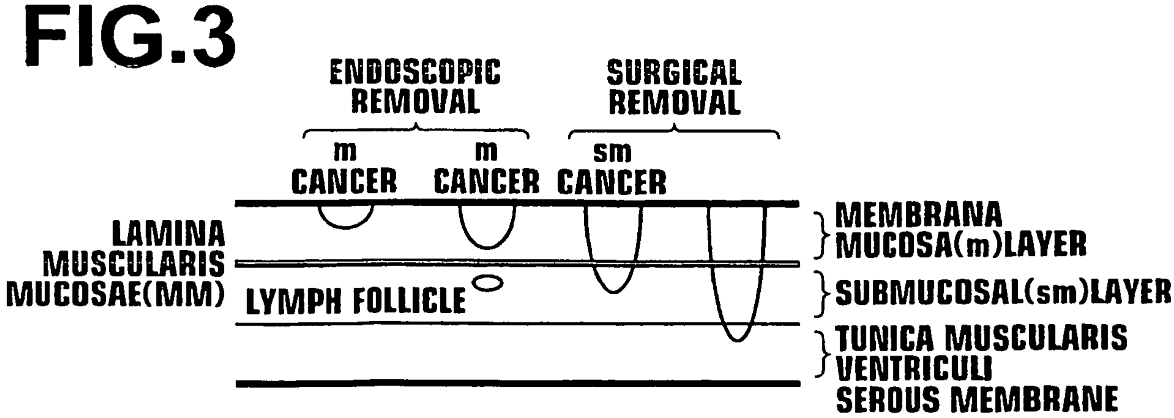
ENDOSCOPIC REMOVAL
SURGICAL REMOVAL
m CANCER
m CANCER
sm CANCER
LAMINA MUSCULARIS MUCOSAE(MM)
LYMPH FOLLICLE
MEMBRANA MUCOSA(m)LAYER
SUBMUCOSAL(sm)LAYER
TUNICA MUSCULARIS VENTRICULI
SEROUS MEMBRANE

FIG.4

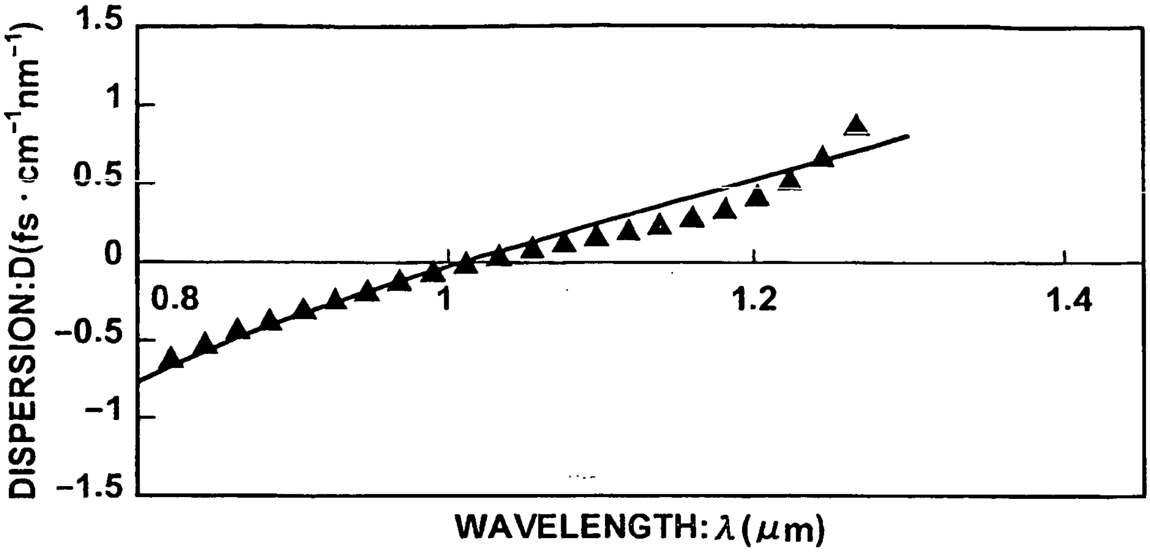
DISPERSION:D(fs · cm⁻¹nm⁻¹)
1.5
1
0.5
0
-0.5
-1
-1.5
0.8
1
1.2
1.4
WAVELENGTH: λ (μm)

FIG.5

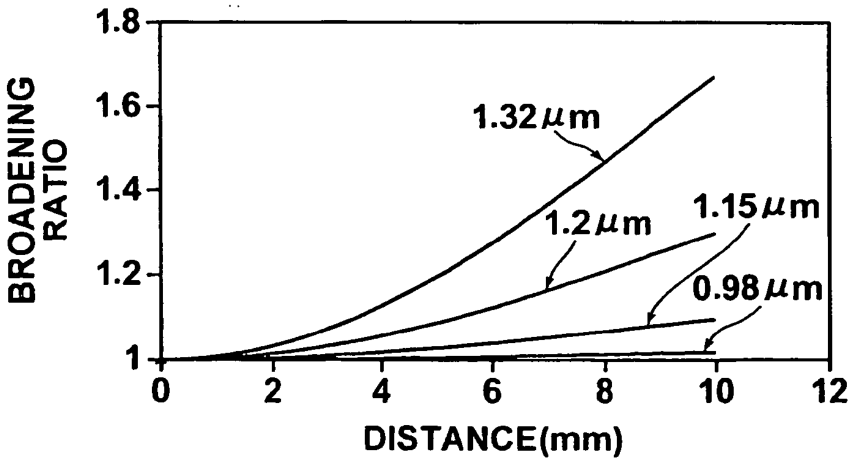
BROADENING RATIO
1.8
1.6
1.4
1.2
1
0
2
4
6
8
10
12
DISTANCE(mm)
1.32μm
1.2μm
1.15μm
0.98μm

FIG.6

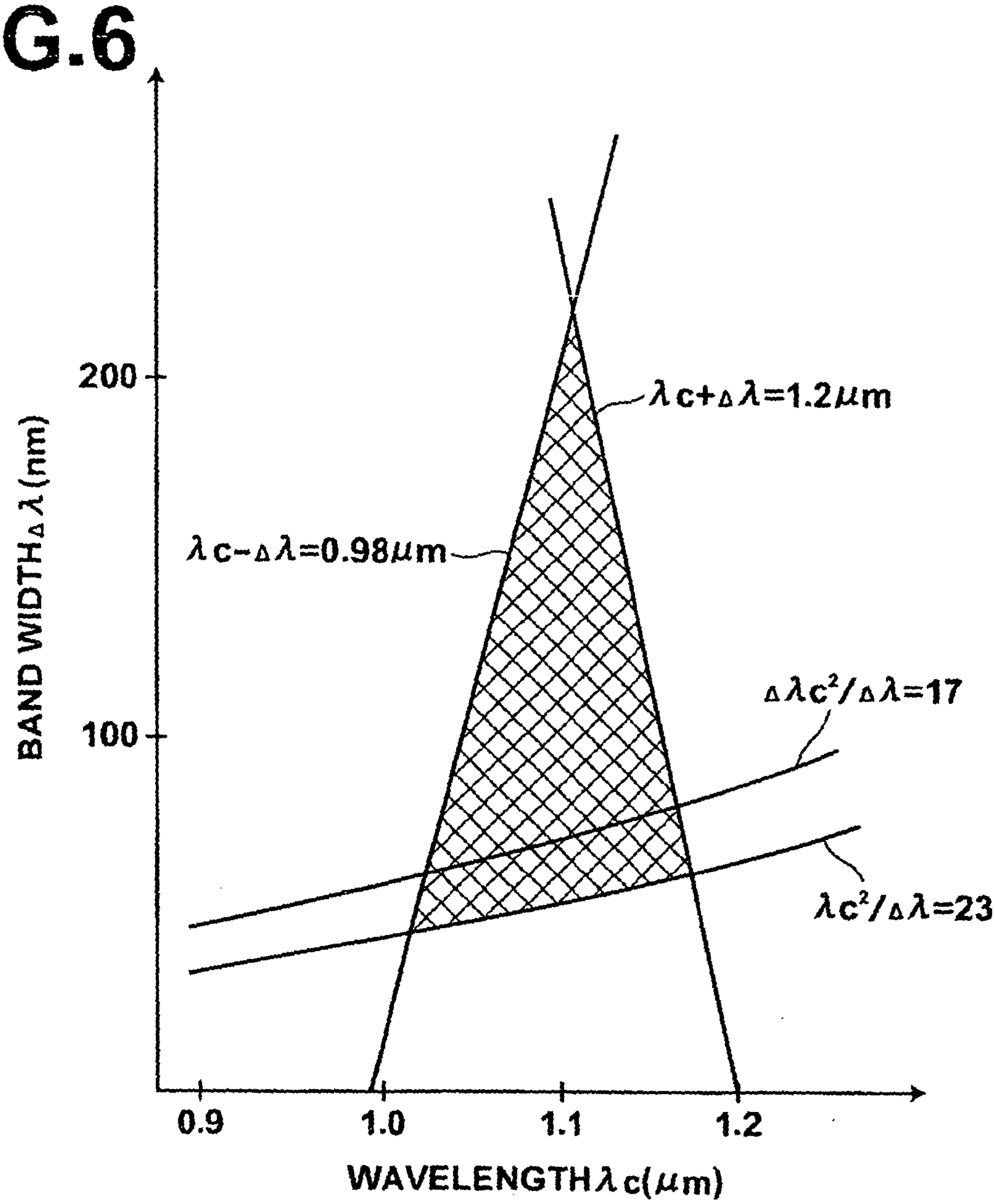
200
100
BAND WIDTH Δλ (nm)
λc+Δλ=1.2μm
λc−Δλ=0.98μm
Δλc²/Δλ=17
λc²/Δλ=23
0.9
1.0
1.1
1.2
WAVELENGTH λc(μm)

FIG.7

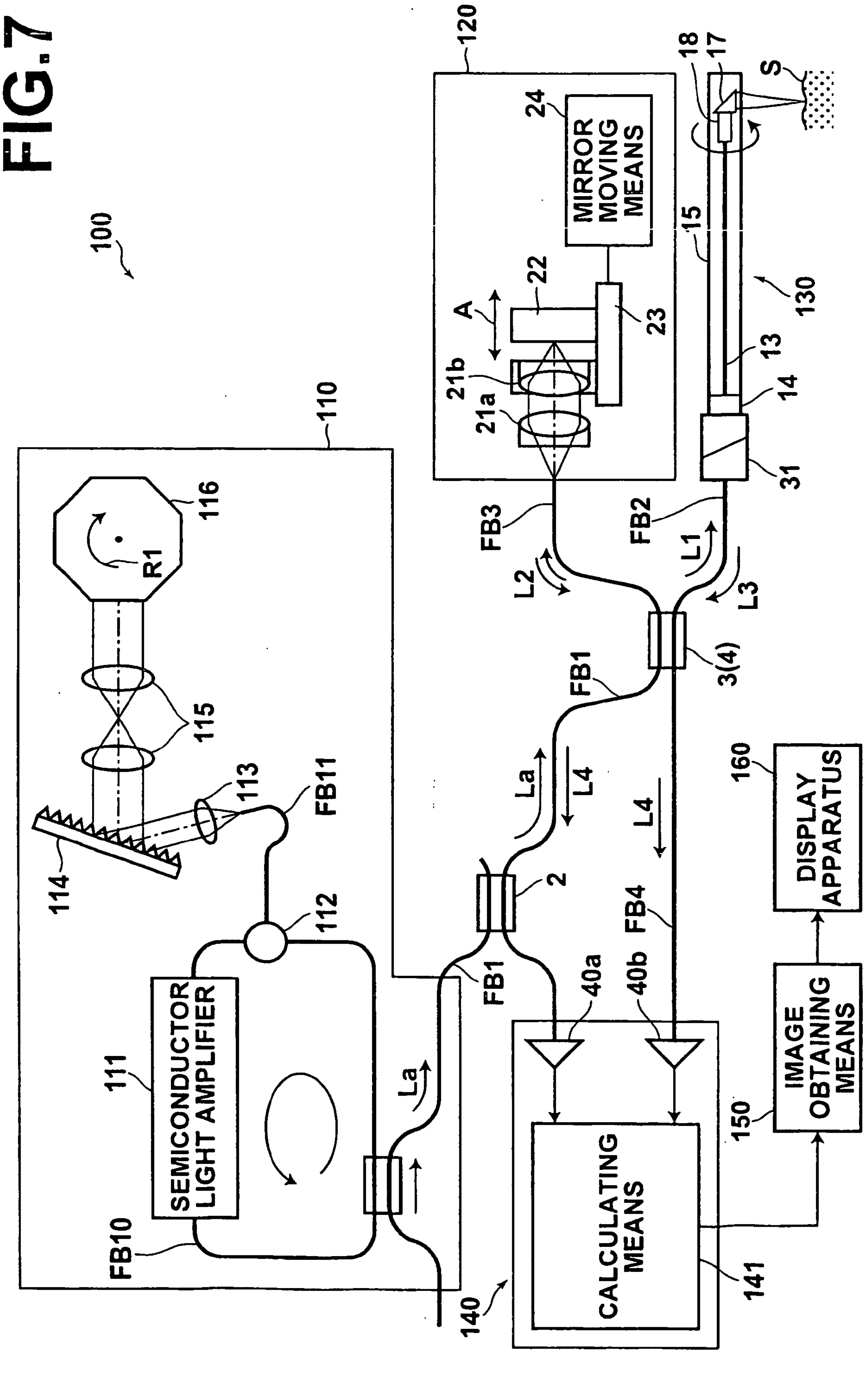
100
110
111
SEMICONDUCTOR LIGHT AMPLIFIER
112
113
114
115
116
R1
FB10
FB11
La
FB1
2
La
L4
FB1
3(4)
L2
L3
L1
FB3
FB2
120
21a
21b
A
22
23
24
MIRROR MOVING MEANS
31
14
13
130
15
18
17
S
L4
FB4
40a
40b
140
CALCULATING MEANS
141
150
IMAGE OBTAINING MEANS
160
DISPLAY APPARATUS

# FIG.8

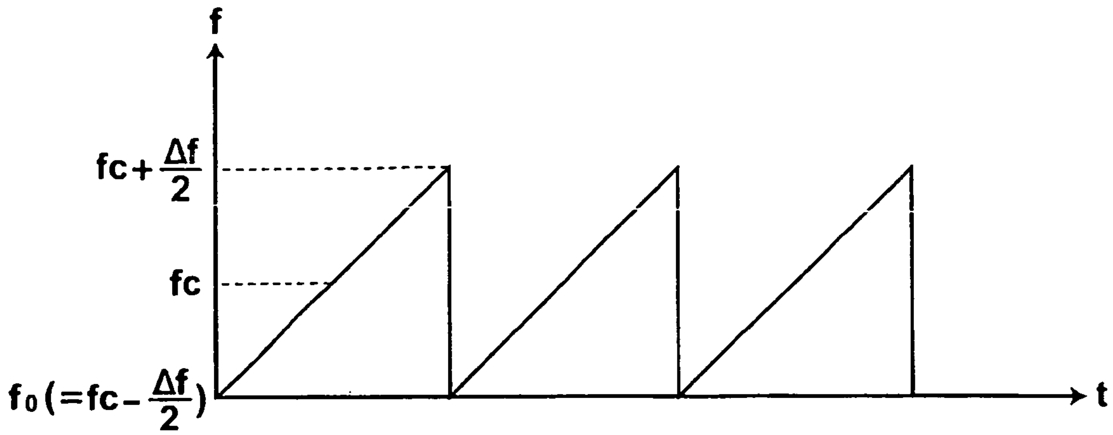
f
fc+Δf/2
fc
f0(=fc-Δf/2)
t

# FIG.9

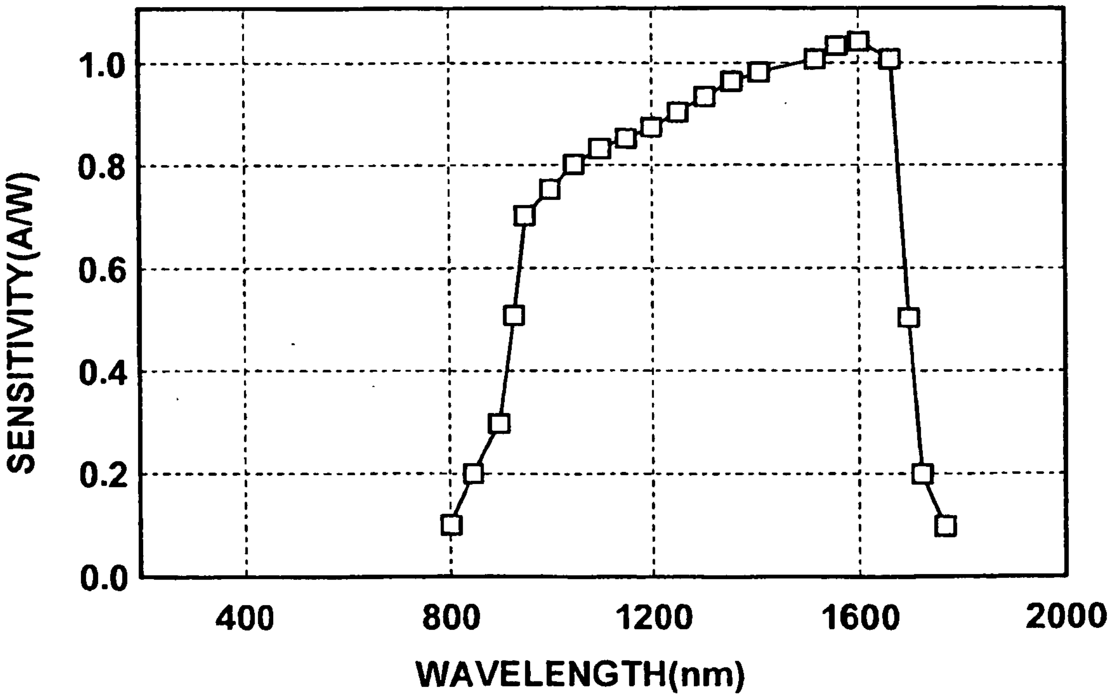
SENSITIVITY(A/W)
1.0
0.8
0.6
0.4
0.2
0.0
400
800
1200
1600
2000
WAVELENGTH(nm)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6165784 A
- JP 2003139688 A
- US 2003055342 A
- US 6728571 B


### Non-patent literature cited in the description


- **W. DREXLER et al.** In Vivo Ultrahigh-Resolution Optical Coherence Tomography. *Optics Letters,* 1999, vol. 24 (17), 1221-1223
- **I. HARTL et al.** Ultrahigh-Resolution Optical Coherence Tomography Using Continuum Generation in an Air-Silica Microstructure Optical Fiber. *Optics Letters,* 2001, vol. 26 (9), 608-610
- **Y. WANG et al.** Optimal Wavelength for Ultrahigh-Resolution Optical Coherence Tomography. *Optics Express,* 2003, vol. 11 (12), 1411-1417
- **M. TAKEDA.** Optical Frequency Scanning Interference Microscopes. *Optical and Electro-Optical Engineering Contact,* 2003, vol. 41 (7), 426-432